## Europäisches Patentamt

## European Patent Office

## Office européen des brevets

(11) Publication number: **0 132 674**
**B1**

# EUROPEAN PATENT SPECIFICATION

(45) Date of publication of patent specification: **19.12.90**

(21) Application number: **84107990.8**

(22) Date of filing: **07.07.84**

(51) Int. Cl.⁵: **A 23 L 1/015,** **C 12 H 1/00,** **C 12 N 9/06**

(54) Amine removal.

(30) Priority: **20.07.83 GB 8319540**

(43) Date of publication of application:
**13.02.85 Bulletin 85/07**

(45) Publication of the grant of the patent:
**19.12.90 Bulletin 90/51**

(84) Designated Contracting States:
**AT BE CH DE FR GB IT LI NL SE**

(56) References cited:
**FR-A-2 434 582**

**FOOD SCIENCE & TECHNOLOGY ABSTRACTS, no. 83040904; J. PAVELKA: "Physical and chemical measures for lowering the histamine level of flesh in fishes of the mackerel family, and possibilities of their application in production of fish-based foods", &**

**The file contains technical information submitted after the application was filed and not included in this specification**

(73) Proprietor: **BOVRIL LIMITED**
**Bovril Limited Beecham House Great West Road Brentford Middlesex TW8 9BD (GB)**

(72) Inventor: **Hobson, John Charles**
**28 Jordan Avenue Stretton Burton-on-Trent Staffordshire DE13 0JA (GB)**
Inventor: **Anderson, Deborah Anne Georgina**
**81 Westmead Road Barton-under-Needwood Staffordshire DE13 8JR (GB)**

(74) Representative: **Russell, Brian John et al Beecham Pharmaceuticals Great Burgh Yew Tree Bottom Road Epsom Surrey KT18 5XQ (GB)**

Courier Press, Leamington Spa, England.

**EP 0 132 674 B1**

(56) References cited:
**VETERINARNI MEDICINA, 27 (4), 237-246, 21, 1982**
**Idem**

**CHEMICAL ABSTRACTS, vol. 63, 1965, column 4587ef, Columbus, Ohio, US; H. YAMADA et al.: "Crystalline amine oxidase of aspergillus niger", & MEM. RES. INST. FOOD. SCI. KYOTO UNIV. NO. 26, 21-3, 1965**

**PATENTS ABSTRACTS OF JAPAN, vol. 7, no. 161 (C-176) 1306r, 15th July 1983; & JP - A - 58 71 896 (KIKKOMAN SHOYU K.K.) 28-04-1983**

## Description

The present invention relates to the enzymatic removal of free amines from foodstuffs and in particular, to the use of amine oxidase enzymes for effecting such removal. As used herein, the term foodstuff is intended to included beverages and solid food material.

Many foodstuffs contain free amines which may be naturally present or may be the products of microbial or synthetic processes, including the products of processes used in the preparation and production of the foodstuffs. Certain of these amines, especially tyramine and phenylethyl amine have been implicated as causing migraines, and for this reason, migraine sufferers are often advised to avoid foodstuffs which have high free amine content, including chocolate, cheese, especially mature cheese, salami, wine and yeast extracts. Furthermore the presence of free amines in the diet can seriously interfere with treatment of depression using mono-amine oxidase inhibitors (MAOI). Patients undergoing MAOI treatment have severely reduced ability to metabolise ingested amines, and a build up of free amines has, in rare instances, even led to death. Again, this problem can only be overcome by avoiding foodstuffs having high levels of free amines.

UK Patent Application 2 031 948 describes the removal of histamine from foodstuffs by enzymatic treatment to destroy histidine, the immediate precursor of histamine. This, however, is not a general method for removing free amines and is directed only at reduction of histamine levels in foodstuffs.

An article by J. Pavelka, *Veterinarni Medicina*, 1982, *27*, 237—346 reports a study undertaken to show the physical and chemical effects on the reduction of histamine content in the muscle of scombroidal fish using an amine oxidase enzyme. The enzyme was however, derived from either an animal or vegetable source and the results obtained were "negligible".

It has now been found that the amount of free amines in foodstuffs may be reduced by treatment with an amine oxidase enzyme at a suitable stage in the production process.

According to the present invention there is, therefore, provided a process for reducing the free amine content of a foodstuff which process comprises contacting the foodstuff with an amine oxidase enzyme derived from the microorganism *Aspergillus niger* at a temperature of between 20°C and 50°C and in a medium at a pH of between 7 to 9, in the presence of molecular oxygen.

Suitable enzymes are mono-amine oxidase and di-amine oxidase, although the latter is preferred due to its wider substrate specificity. Such enzymes catalyse the oxidative deamination of free amines to aldehydes.

This reaction may be performed under a variety of conditions provided that extremes of pH and temperature and ion concentration are avoided. In particular, the conditions should be such that the enzyme is not denatured or degraded for a period sufficient for the enzyme to catalyse the desired reaction.

For any particular enzyme, the exact conditions giving optimum performance will be readily determinable by the skilled person.

It has been found to be convenient to use temperatures in the range from 10 to 70°C, preferably 20 to 50°C, for example 35°C, and a pH in the range from 2 to 10, preferably 7 to 9, for example pH 8.

Amine oxidase enzymes require, in addition to the free amine, free molecular oxygen. Normally there will be sufficient oxygen in the foodstuff to be treated, however, in certain cases it may be found desirable to add extra oxygen.

Mono- and di-amine oxidases may be obtained from a variety of sources, for instance, mammalian tissue and from micro-organisms. It has been found particularly convenient to obtain di-amine oxidase from organisms of the genus *Aspergillus*, in particular from *Aspergillus niger,* for the strain of *A. niger* desposited with the Commonwealth Mycological Institute under accession number IMI 17454. The enzyme from this latter organism is preferred because of its wide substrate specificity. Substrates oxidised include tyramine, phenylethylamine, tryptamine, n-butylamine, putrescine, benzylamine and histamine. The enzyme has been shown to contain both copper and pyridoxal phosphate and is therefore broadly classified, as the 1.4.3.6. type according to internationally recognised nomenclature. However, the enzyme may be obtained from other strains and species, and from other micro-organisms. Another source of di-amine oxidase has been found to be a yeast, *Saccharomycopsis lipolytica*.

These enzymes have been found to have a range of substrates corresponding approximately with that of human amine oxidases and thus are especially useful in destroying free amines which would otherwise interfere in MAOI treatment.

The enzyme 'histaminase' is also useful in the present invention, not only in reducing histamine content but in reducing the content of other free amines, since this name is apparently incorrect and this enzyme is actually a di-amine oxidase.

The enzyme may be obtained by culturing the selected organism in a culture medium containing free amines, which induce the production of the enzyme. When a suitable degree of growth has occurred the enzyme may be harvested from the culture medium if secreted by the micro-organism, or by disrupting the cells, and extracting the enzyme from the resultant solution by conventional means. Such a procedure is described, for instance, by Yamada *et al., Agr. Biol. Chem.,* 29 (7), 649—654, (1965).

The enzyme may be used to treat the foodstuff at any suitable stage in the production thereof. Conveniently the enzyme may be added at a stage when the foodstuff is in liquid form, preferably in a moderately concentrated form, but not so concentrated that the enzyme is denatured or precipitated before oxidation of the free amines has occurred. As mentioned above, the conditions of temperature and pH should also be suitable for the enzymatic reaction to occur. The pH and temperature may, if necessary, be adjusted to give suitable conditions, and then readjusted, if necessary, before the production process is continued.

The enzyme in solution may be stabilised using a basic stabiliser, for example, ammonium sulphate or sorbitol, preferably sorbitol. Preservatives may also be added, for example, benzoic acid, hydroxybenzoic acids and potassium sorbate.

As an alternative to, or in addition to the use of the enzyme in solution, the foodstuff may be treated with the enzyme immobilised on an insoluble support means. Such supports for immobilised enzymes are well known and are used conventionally. The enzymes may be attached to the support by conventional physical or chemical methods, such as those described by Karube et al., Enzyme and Microb. Technol. 2, 117, (1980).

The exact means by which the foodstuff is treated with the enzyme will depend on the nature of the production process involved and the optimum will be readily determinable in each particular instance.

As mentioned above, the amine oxidase enzymes may be used to reduce the free amine content of foodstuffs. Whether the free amines are completely removed will depend on the length of time for which the foodstuff is exposed to the enzyme. Thus the free amine content can be adjusted to a suitable level by selecting the period of reaction.

It should be noted that in certain cases the free amine content of a foodstuff may be reduced, or the free amines completely removed, but that further free amines may be generated in a later stage of the production process. This is especially true of matured cheese in which the maturation produces free amines, and indeed these may be in part responsible for the taste and smell of matured cheese.

Once the enzymatic catalysis is complete, or has reached a satisfactory stage, the reaction may be stopped by denaturing the enzyme. As most foodstuff production processes include a heat sterilisation stage late in the process, this will be sufficient to denature the enzyme. Whilst it is desirable that the enzyme be denatured, it is not essential. Preliminary toxicological testing has indicated that the enzyme is not detrimental and is safe to be included in foodstuffs.

In a particular embodiment of the present invention, there is provided a process for reducing the free amine content of yeast extracts which process comprises contacting yeast extract, at a suitable stage in the production thereof, with an amine oxidase enzyme and then subsequently denaturing the enzyme.

In cheese making, an amine oxidase may conveniently be added during the early stages of the process when other enzyme treatments for improving the flavour are often applied. Treatment at later stages may be restricted because of the lack of sufficient oxygen. This applies particularly to matured cheese undergoing a long ripening period.

In beer and wine making, the treatment may be applied before fermentation when there is sufficient dissolved oxygen present.

In certain cases, the conditions in the various stages of manufacture of a foodstuff may be unfavourable to the use of an amine oxidase enzyme, according to the process of the present invention, and it may be impracticable, or deleterious to the foodstuff, to adjust the conditions to those required. In such cases it may be possible to use an immobilised amine oxidase, as hereinbefore described, as immobilisation may render the enzyme more stable to extremes of temperature, pH or ion concentration.

The production of yeast extract involves the steps of disrupting the cells, removing the cell debris and concentrating and treating the liquors. The enzymatic destruction of free amines is suitably performed at an intermediate stage in the process of concentrating the extract. A suitable purified enzyme, such as that extracted from Aspergillus niger (IMI 17454), is added after the pH and temperature have been adjusted to the aforementioned ranges and, if necessary, the dissolved oxygen content of the extract has been adjusted to a suitable level. Conveniently the extract is allowed to stand for a period of a few hours to a few days to allow the enzymatic reaction to proceed. The enzyme is later denatured by heating stages in the latter part of the yeast extract production process.

Alternatively the extract may be allowed to flow over or through a bed of immobilised enzyme. In this case the process may be run continuously rather than as a batch process when enzyme is added directly to the extract. If run as a continuous process, the flow rate of the extract must be adjusted to allow sufficient time for the enzymatic reaction to occur.

A similar procedure may be adopted during the processing of other foodstuffs, such as wines, beer, fish, cheese, milk products, chocolate and meat pastes. In each case the enzyme is added to a liquid stage of the procedure, and when applied to fish, cheese and meat pastes, is preferably added when the solids are in a comminuted form such that a large surface area of the solids is exposed to the enzyme.

In treating such solids it may not be possible to achieve complete removal of free amines because the interior of the particles are not necessarily accessible to the enzyme. However, it should be understood that any reduction in the free amine content is considered to be an advantage.

In the case of beer making, the treatment is preferably applied to the wort, the medium which is fermented by yeast to produce beer. For wort production, barley is first steeped in several changes of water until germination commences. After a few days germination is arrested and the material is dried under controlled conditions to give malt. The malt is then ground to a fine powder, water is added and heat applied using a defined temperature treatment. This is known as the 'mashing' stage, during which malt enzymes degrade carbohydrate and protein polymers. Following filtration, hops are added and the wort is boiled and thus sterilised. After the completion of the boil, the hot wort is pumped through a strainer to remove residues and then allowed to cool prior to addition of pitching yeast.

Treatment of wort rather than beer is less likely to cause undesirable flavour changes caused by temperature, pH and maximal levels of dissolved oxygen. Treatment of the wort after the boiling stage will, however, be conducive towards the survival of active amine oxidase through to finished beer. If this is found to be a problem, the enzyme could be applied at the mashing stage, although the conditions at this point would, of course, be less suitable for maximal amine oxidase activity.

The present invention will now be illustrated with reference to the following Examples, which are not intended to limit the invention in any way.

Example 1

Production of Enzyme

*Aspergillus niger* (IMI 17454) was maintained on potato dextrose agar (PDA) slopes stored at 4°C and renewed every month. The organism was cultured as follows:

Spores from a PDA slope were suspended in nitrate medium (infra) (10 ml) and the suspension used to inoculate culture flasks containing nitrate medium (150 ml each). The flasks were incubated for 24 hours at 20°C with reciprocal shaking. The subculture was inoculated into jar fermentors containing nitrate medium (8 l) and incubated at 20°C for 24 hours with aeration. The mycelial growth was harvested using a sterile Buchner flask and funnel and transferred into Braun fermentors containing n-butylamine medium (infra) (8 l). The fermentors were maintained at 30°C with an air flow of 1 l/min/l medium and agitated (400 rpm). The foam level in the fermentors was controlled by automated addition of silicone antifoam (sterile) and the pH maintained in the range pH 5.0 to 5.8 by automated addition of n-butylamine (1.0 M). After 17 hours growth in the n-butylamine medium cells were harvested by filtration.

Isolation of Enzyme

The following steps were conducted at about 5°C.

The mycelia harvested from the n-butylamine medium were washed (distilled water) at 5°C. The filter cake was ground with sea sand in a chilled mortar for about 5 minutes. The product was suspended in phosphate buffer (0.01 M, pH 7.0) and centrifuged (6000 × g for 10 mins) to remove cell debris and sea sand. The supernatant was dialysed against phosphate buffer (0.005 M, pH 7.0, 10 l) for 24 hours. The dialysate served as crude enzyme preparation.

The enzyme was further purified by conventional means when necessary, and may be obtained in crystalline form, however, in practice, only relatively crude enzymes would be used on a commercial scale.

Culture Media

"nitrate medium"

|  | % w/v |
| --- | --- |
| Glucose | 3.0 |
| Dipotassium hydrogen phosphate | 0.1 |
| Magnesium sulphate | 0.05 |
| Potassium chloride | 0.05 |
| Sodium nitrate | 0.1 |
| Distilled water | to 100 |

"n-butylamine medium"

As for "nitrate medium" but with n-butylamine 1% w/v, in place of sodium nitrate.

All media were adjusted to pH 6.0 and sterilised prior to use.

Example 2

Reduction of free amine content of yeast extract — batch process

Yeast extract was prepared by autolysis at elevated temperature, then separating the cell debris by sieving and using centrifugal separators. The separator liquors afforded a solution containing 6—8% refractories (dissolved solids). The liquor was cooled to 35°C, the pH adjusted to pH 7.5 to 8.0 by addition of alkali and the purified diamine oxidase from *A. niger* (IMI 17454) was added at $4 \times 10^4$ enzyme units per litre of extract. The reaction was allowed to proceed for two hours. The extract was then further cooled, evaporated, settled and filtered and further evaporated to afford a concentrated yeast extract.

Amine levels were assayed by hplc as described below. The results are as follows:

|  | Tyramine |
|---|---|
| Enzyme treated yeast extract | none detected |
| Untreated yeast extract | 441 µg/g |

A qualitative reduction in histamine levels has also been observed.

Example 3

Reduction of free amine content of Beer

Enzyme application process

Three wort samples differing in specific gravity and total nitrogen content were evaluated. For each wort sample, the effect of incubation pH was tested over the following range:

(i) The natural pH of the wort [pH 5.0—5.6]

(ii) Adjusted to pH 6.0 [using 0.4N NaOH]

(iii) Adjusted to pH 7.5 [the optimum pH for amine oxidase application in yeast extract process separator liquor]

In addition, since the tyramine content of beer is known to be very low compared with that of separator liquor, samples containing additional tyramine to 50 µg/ml were also prepared at the above pH values. Thus, the effectiveness of amine oxidase activity in wort at unusually high tyramine levels could be evaluated, and also the recoveries calculated in order that correction factors could be applied.

In each case, 100 ml of wort was placed in a culture flask [250 ml] and enzyme added at the rate of 40 units/ml. The treatment of wort [1] involved a dosage rate of 80 units/ml at the natural pH since it was envisaged that such a low pH would severely depress enzyme activity. All further applications were carried out at 40 units/ml.

Following enzyme addition, the flasks were incubated at 35°C with orbital shaking in order to ensure maximum dissolved oxygen availability. Samples were removed after 0 and 4 hours incubation.

Amine assay

Samples were extracted and the amines quantified according to the standard procedure applied to yeast extract process separator liquor (see appendix).

Efficiency of amine oxidase activity

Details of the three worts studied are presented in Table 1. The following observations can be made:

(i) Tyramine levels in the untreated worts ranged from 0.801 to 2.229 µg/ml.

(ii) It is clearly apparent that, in the range tested, pH 7.5 is optimal for amine oxidase activity in wort and ensures efficient oxidation at elevated tyramine levels.

(iii) Unsupplemented tyramine levels were maximally oxidased after 4 hours incubaton at the natural wort pH.

Average figures for the three worts are shown in Table II and confirm the above indications. In addition, it is also apparent that tyramine levels of around 50 µg/ml were dependent on a pH of 7.5 for complete oxidation after 4 hours incubation.

No histamine was detected in any of the worts assayed.

At tyramine levels of up to 2 µg/ml, amine oxidase activity at a dosage rate of 40 units/ml is sufficient for complete oxidation at the natural wort pH after 4 hours incubation under defined conditions. This therefore raises the possibility that brewers may be able to utilise amine oxidase without the necessity for a pH adjustment step.

In this study, enzyme activity was found to be most efficient at pH 7.5. Thus, on large scale application, adjustment to this pH would ensure minimal enzyme requirement and/or treatment time.

Amine oxidase from *Aspergillus niger* I.M.I/ 17454, when applied to aerated wort under defined conditions, is effective in reducing the tyramine content through oxidation.

For natural tyramine levels of up to 2 µg/ml, an enzyme dosage rate of 40 units/ml wort is sufficient together with agitated incubation for 4 hours at 35°C. The minimal enzyme dosage rate under these conditions may well be much less than 40 units/ml.

Highly efficient tyramine oxidation could be guaranteed by pH adjustment to 7.5 which would minimise both enzyme dosage rate and incubation time.

# EP 0 132 674 B1

## TABLE [1]
### Effect of Amine Oxidase Application on the Tyramine Content of Three Wort Types

*Tyramine added to 50 µg/ml        ND: None Detected

Wort [1]
pH: 5.0 Total Nitrogen: 675.0 mg/l
Specific Gravity: 1.0448   Solids Content: 11.48% w/v
Enzyme Dosage Rate:  80 units/ml [pH 5.0]
                     40 units/ml [pH 6.0]
                     40 units/ml [pH 7.5]

| Incubation pH | Tyramine Concentration [µg/ml] | |
|---|---|---|
| | 0 Hr | 4Hr |
| 5.0 | 1.400 | ND |
| 5.0* | 51.360 | 24.32 |
| 6.0 | 1.746 | ND |
| 6.0* | 51.690 | ND |
| 7.5 | 1.215 | ND |
| 7.5* | 51.310 | ND |

Wort [2]
pH: 5.55 Total Nitrogen: 616.3 mg/l
Specific Gravity: 1.0365   Solids Content: 9.16% w/v
Enzyme Dosage Rate: 40 units/ml throughout

| Incubation pH | Tyramine Concentration [µg/ml] | |
|---|---|---|
| | 0 Hr | 4Hr |
| 5.55 | 2.229 | ND |
| 5.55* | 52.240 | ND |
| 6.0 | 1.449 | ND |
| 6.0* | 51.420 | ND |
| 7.5 | 1.005 | ND |
| 7.5* | 50.860 | ND |

7

Wort [3]
pH: 5.60 Total Nitrogen: 804.2 mg/l
Specific Gravity: 1.0319   Solids Content: 8.20% w/v
Enzyme Dosage Rate:  40 units/ml throughout

| Incubation pH | Tyramine Concentration [µg/ml] | |
|---|---|---|
| | 0 Hr | 4Hr |
| 5.6 | 0.801 | ND |
| 5.6* | 50.860 | 23.94 |
| 6.0 | 0.616 | ND |
| 6.0* | 47.56 | 25.45 |
| 7.5 | 0.886 | ND |
| 7.5* | 50.81 | ND |

TABLE [2]
Average Figures
*Tyramine added to 50 µg/ml

| Incubation pH | Tyramine Concentration [µg/ml] | |
|---|---|---|
| | 0 Hr | 4Hr |
| 5.38 | 1.48 | 0 |
| 5.38 | 51.49 | 16.09 |
| 6.0 | 1.270 | 0 |
| 6.0* | 50.22 | 8.48 |
| 7.5 | 1.035 | 0 |
| 7.5* | 50.99 | 0 |

Appendix
Hplc assay for free amines
The method is based on that of Mell et al. J. Liquid Chrom. 1 (3) 261—277 (1978).
Foodstuffs are assayed for free amines as follows:
o-Phthalaldehyde derivatising reagent was prepared by dissolving o-phthalaldehyde (160 mg) in ethanol (3 cm³) and 2-mercaptoethanol (0.2 cm³). This solution was added to boric acid (0.4 $M$, 100 cm³) which had been adjusted to pH 9.5 with potassium hydroxide. (This reagent is stored at 4°C and has a shelf life of 1 week).
Extracted and purified samples of foodstuff, or beverage (0.4 cm³) were treated with derivatising reagent (0.5 cm³) and of serine (0.1 cm³) (20 µg/cm³ in 1 $M$ hydrochloric acid and 4 $M$ sodium hydroxide, pH 6.5). The mixture was allowed to react for 2 minutes then 10 µl aliquots were injected into the HPLC machines.
Column type — Reversed phase C8 bonded silica 5 µm average particle size.
Dimensions — 4 mm i.d. × 25 cm
Temperature — ambient
Flow rate — 1.3 ml/min
Solvent — methanol/1% heptane sulphonic acid (60/40 by vol)
Serine serves as an internal standard.
A standard amine solution was made up as for the above sample containing 5 µg/cm³ of each amine to be assayed in place of the homogenised foodstuff or beverage.

Amine content was calculated as follows:

$$\text{amine conc } \mu g/cm3 = \frac{\left(\dfrac{\text{Peak area amine}}{\text{peak area int. stand.}}\right)_{sample}}{\left(\dfrac{\text{Peak area amine}}{\text{peak area int. stand.}}\right)_{standard}} \times \frac{\mu g \text{ amine in standard}}{\text{sample volume } (cm^3)}$$

## Claims

1. A process for reducing the free amine content of a foodstuff with an amine oxidase which process comprises contacting the foodstuff with an amine oxidase enzyme derived from the microorganism *Aspergillus niger* at a temperature of between 20 to 50°C and in a medium at a pH of between 7 to 9, in the presence of molecular oxygen.

2. A process according to claim 1 wherein the amine oxidase is a di-amine oxidase of the 1.4.3.6. type.

3. A process according to claim 1 or claim 2, wherein the *Aspergillus niger* organism is the strain deposited with the Commonwealth Mycological Institute under accession number 1M1 17454.

4. A process according to any one of claims 1 to 3 wherein the amine oxidase is subsequently denatured.

5. A process according to any one of claims 1 to 4 wherein the amine oxidase is in a solution also comprising a basic stabilizer.

6. A process according to claim 5, wherein the amine oxidase solution also comprises a preservative.

7. A process according to any one of claims 1 to 7, wherein the foodstuff is a yeast extract.

## Patentansprüche

1. Ein Verfahren zur Reduktion des Gehalts an freiem Amin eines Lebensmittels mit einer Aminoxidase, welches Verfahren das Kontaktieren des Lebensmittels mit einem Aminoxidaseenzym, das aus dem Mikroorganismus *Aspergillus niger* gewonnen wurde, bei einer Temperatur von zwischen 20 und 50°C und in einem Medium bei einem pH-Wert von 7 bis 9, in Gegenwart von molekularem Sauerstoff umfaßt.

2. Ein Verfahren nach Anspruch 1, in welchem die Aminoxidase eine Diaminoxidase des 1.4.3.6-Typs ist.

3. Ein Verfahren nach Anspruch 1 oder 2, in welchem der *Aspergillus niger*- Organismus der Stamm ist, welcher beim Commonwealth Mycological Institute unter der Nummer 1M1 17454 hinterleft ist.

4. Ein Verfahren nach einem der Ansprüche 1 bis 3, in welchem die Aminoxidase anschließend denaturiert wird.

5. Ein Verfahren nach einem der Ansprüche 1 bis 4, in welchem die Aminoxidase in einer Lösung vorliegt, welche außerdem einen basischen Stabilisator enthält.

6. Ein Verfahren nach Anspruch 5, in welchem die Aminoxidaselösung außerdem ein Konservierungsmittel enthält.

7. Ein Verfahren nach einem der Ansprüche 1 bis 7, in welchem das Lebensmittel ein Hefeextrakt ist.

## Revendications

1. Procédé pour réduire la teneur en amine libre d'une denrée alimentaire avec une amine oxydase, caractérisé en ce qu'il comprend la mise en contact de la denrée alimentaire avec une enzyme amine oxydase dérivée du microorganisme *Aspergillus niger* à une température comprise entre 20 et 50°C et dans un milieu à un pH compris entre 7 et 9 en présence d'oxygène moléculaire.

2. Procédé suivant la revendication 1, caractérisé en ce que l'amine oxydase est une di-amine oxydase du type 1,4,3,6.

3. Procédé suivant la revendication 1 ou la revendication 2, caractérisé en ce que l'organisme *Aspergillus niger* est la souche déposée par the Commonwealth Mycological Institute sous le numéro d'accès 1M1 17454.

4. Procédé suivant l'une quelconque des revendications 1 à 3, caractérisé en ce que l'amine oxydase est ensuite dénaturée.

5. Procédé suivant l'une quelconque des revendications 1 à 4, caractérisé en ce que l'amine oxydase est dans une solution contenant aussi un stabilisant basique.

6. Procédé suivant la revendication 5, caractérisé en ce que la solution d'amine oxydase comprend aussi un préservatif.

7. Procédé suivant l'une quelconque des revendications 1 à 6, caractérisé en ce que la denrée alimentaire est un extrait de levure.